# EUROPEAN PATENT APPLICATION

(11) **EP 1 681 036 A1**
(43) Date of publication of application: **19.07.2006**
(21) Application number: 06100364.6
(22) Date of filing: 16.01.2006
(51) Int. Cl.: A61F 2/34

(54) **Perfected acetabular cup prosthesis for a hip**

(30) Priority: 17.01.2005 IT MI20050038
(71) Applicant: PIERANNUNZII, Luca Massimo Carlo, I-20126 Milano (IT)
(72) Inventor: PIERANNUNZII, Luca Massimo Carlo, I-20126 Milano (IT)
(74) Representative: Petraz, Davide Luigi

(57) **Abstract**

Acetabulum cup (10) for arthro-prosthesis of the hip comprising an inner surface (12) of a hemispherical conformation and a shaped edge (13), which edge (13) has an anatomical and asymmetric profile, different for the left and right sides with respect to a vertical diameter thereof (Z), so that an angle of ante-version (ß) defined by said edge (13) varies from an upper end (Ps) to a lower end (Pi) of said vertical diameter (Z).

## Description

### FIELD OF THE INVENTION

The present invention consists of a perfected acetabulum cup for the arthro-prosthesis of a hip. It is therefore intended for use in degenerative or traumatic pathologies of the articulation of the human hip.

### BACKGROUND OF THE INVENTION

The state of the art provides that the acetabulum cup for the arthro-prosthesis of the hip, hereafter called more simply acetabulum (prosthetic) cup, has an inner surface shaped like a spherical cap, with a subtended plane angle equal to 180° (hemisphere) or less. The edge of the cap is circular, and can at most have focal protrusions or reductions, normally symmetrical, in correspondence with a limited sector of the cup. The cup can be a single block or modular, in this case consisting of two components, a metal cup outside (intended for contact with the pelvis bone) and a concave insert inside (intended to cooperate with the head of the prosthesis).

To obtain an articular travel as close as possible to a natural hip, the traditional cup must be positioned in the pelvis in such a manner that its aperture is facing in a lateral-frontal-lower direction.

To be more exact, the aperture of a known cup, when correctly implanted, describes, on the front plane of the body with respect to the transverse plane, an acute angle open laterally of about 45°, called the angle of inclination. The known cup, when correctly implanted, also describes on the transverse plane, with respect to the sagittal plane, a constant acute angle open to the front of about 15-20°, called the angle of ante-version.

Recent research has shown that, in nature, the acetabulum does not have a constant angle of ante-version, but one progressively increasing from the upper end of the edge (upper pole) to the lower end (lower pole). These studies have also revealed that the angle subtended by the cavity is not the same in all directions, but has its maximum value along the upper/lower direction (on average less than or equal to 180°), while it has progressively decreasing values along the front-upper/rear-lower, front/rear and front-lower/rear-upper directions.

From this it can be deduced that the implantation of a traditional prosthetic acetabulum cup, having a constant and uniform angle of ante-version, entails various disadvantages, including
- the impossibility of restoring a physiological articulation, which cannot be guaranteed by an acetabulum with a uniform ante-version, whatever the angle of ante-version imparted by the surgeon. In fact, both with a small angle of ante-version, and also with a big angle of ante-version, but constant, the neck of the femur or prosthesis knocks, in the movements of flexion, or of extension, respectively, against the front or rear edge of the acetabulum, with the production of detritus, the risk of a mechanical mobilization of the components and rear or frontal dislocation of the implant, to which the insufficient rear or front-upper cover of the head contributes, inversely;
- the need to mill the cavity deeply, removing healthy and vital bone tissue, to prevent part of the prosthetic component from remaining uncovered;
   and
- the risk of developing tendonitis of the iliopsoas due to friction with the front-lower edge of the cup.

Acetabulum cups are also known, from the US patent US-B-6,503,281, provided with a symmetrical recess in which the tendon can slide. Although they reduce the risk of tendonitis of the iliopsoas, such cups do not in any case allow to restore a physiological travel of the joint, and in any case need depth milling to be completely housed.

One purpose of the invention is to obtain an acetabulum cup which, reproducing in the most faithful possible way the anatomy of the normal human acetabulum, guarantees an extensive articular travel, without conflict and interference with the neck of the femur or prosthesis, does not require excessive milling of the cotyloid cavity, and which reduces the risk of tendonitis of the iliopsoas.

The Applicant has devised, tested and embodied the present invention to overcome the shortcomings of the state of the art and to obtain these and other purposes and advantages.

### SUMMARY OF THE INVENTION

The present invention is set forth and characterized in the main claim, while the dependent claims describe other characteristics of the invention or variants to the main inventive idea.

In accordance with the above purpose, the present invention consists of a prosthetic acetabulum cup with an inner surface of spherical conformation, and having an edge shaped in an anatomic and asymmetrical form, different for the right side and the left side with respect to a vertical diameter, wherein the angle of ante-version (as defined above) varies from an upper pole, defined as the upper end of the vertical diameter, to a lower pole, defined as the lower end of the vertical diameter, irrespective of the implant orientation of the cup imparted by the surgeon.

Advantageously, in one embodiment of the invention, the angle of ante-version progressively grows in a substantially continuous manner, going from the upper end to the lower end of the diameter.

According to a variant, the angle of ante-version grows along segments of said vertical diameter, alternating with segments where said angle remains substantially constant.

Geometrically, the sections normal for said vertical diameter, made by proceeding from the upper pole to the lower pole, identify, in the intersection with the edge of the cup, arches whose subtended chord is rotated in an anti-clockwise direction for the right cup, and clockwise for the left cup.

In this way, an asymmetric edge is defined, with a variable angle of ante-version, and mainly increasing, along the diameter so as to reproduce in the most faithful possible way the anatomy of the natural acetabulum, so as to guarantee an effective restoration of a physiological articulation.

In fact, with the present invention, during the movements of flexion and extension, the neck of the prosthesis or femur does not knock against the front or rear edge of the artificial acetabulum, and therefore the production of detritus, the risk of mechanical mobilization of the components and the risk of dislocations are reduced.

Moreover, with the prosthetic acetabulum cup according to the present invention, there is no need to mill the cavity deeply, leaving the healthy and vital bone tissue intact, nor is there the risk of developing tendonitis of the ileopsoas due to friction with the edge of the cup.

### DESCRIPTION OF THE DRAWINGS

In order to demonstrate the characteristics of the invention better, we shall now describe a preferential constructional example. Schematic and non-restrictive drawings of the invention are attached, wherein:
Fig. 1 is a three-dimensional view of a prosthetic acetabulum cup, right, according to the present invention, housed in a pelvis, and where the progressive variation (growth) can be seen of the angle of ante-version from the top downwards;
Fig. 2 is a three-dimensional view of a left prosthetic acetabulum cup according to the present invention;
Fig. 3 is a view from a front plane of the left prosthetic acetabulum cup in fig. 2;
Fig. 4 is a view from a front plane of the right prosthetic acetabulum cup in fig. 1;
Fig. 5 is a view from V of the left prosthetic acetabulum cup in fig. 2;
Fig. 6 is a cross section along the line VI-VI of the left prosthetic acetabulum cup in fig. 2;
Fig. 7 is a cross section along the line VII-VII of the left prosthetic acetabulum cup in fig. 2.

### DETAILED DESCRIPTION OF A PREFERENTIAL FORM OF EMBODIMENT

With reference to the attached figures, a prosthetic acetabulum cup 10 according to the present invention substantially comprises a cap 11, hemispherical in shape, which subtends a plane angle less than or equal to 180°, preferably comprised between 140° and 180°, and in the example equal to 172°. The cap 11 has an inner surface 12, also hemispherical in shape, and an edge 13 shaped in an anatomic form. The inner surface 12 and the edge 13 thus define a cavity 14 open towards the outside and in which the natural or artificial head of the femur is able to be housed.

To be more exact, the shaping of the edge 13 is asymmetrical with respect to its own diameter Z, or vertical diameter, passing through the center of the aperture of the cavity 14, that is, the cup 10 defines a variable angle of ante-version β, that is, growing over the length of the diameter Z.

You should consider that β is an angle of ante-version that depends exclusively on the particular shape of the invention and not on the positioning choice made by the surgeon, which can and must orient the acetabulum cup appropriately, according to the case and his experience. From this point the angle βwill de defined only as the angle of ante-version.

In the embodiment shown here, the angle of ante-version grows progressively and continuously going from the upper end to the lower end of the vertical diameter Z.

However, it comes within the field of the present invention that segments which have a growth of the angle of ante-version are separated by segments in which said angle remains substantially constant.

According to the invention, the shaping is geometrically obtained by identifying, first of all, two opposite points Ps and Pi, in correspondence with the zones of intersection or end zones of the vertical diameter Z with the edge 13, which will now be defined arbitrarily as upper pole (Pₛ) and lower pole (Pᵢ) . We define the segment PₛPᵢ as an axis of roto-translation, coinciding with the vertical diameter Z.

Now we shall section the cap 11 with a straight line r, orthogonal to the vertical diameter Z, which moves along the latter proceeding from Pₛ towards Pᵢ, preferably with a uniform rectilinear movement.

The straight line r, during this translation, also performs a movement of rotation around the vertical diameter Z, such that in correspondence with Pₛ it describes with the plane of aperture of the spherical cap 11 an angle αₛ (advantageously zero), while in correspondence with Pᵢ it describes with said reference plane an angle αᵢ.

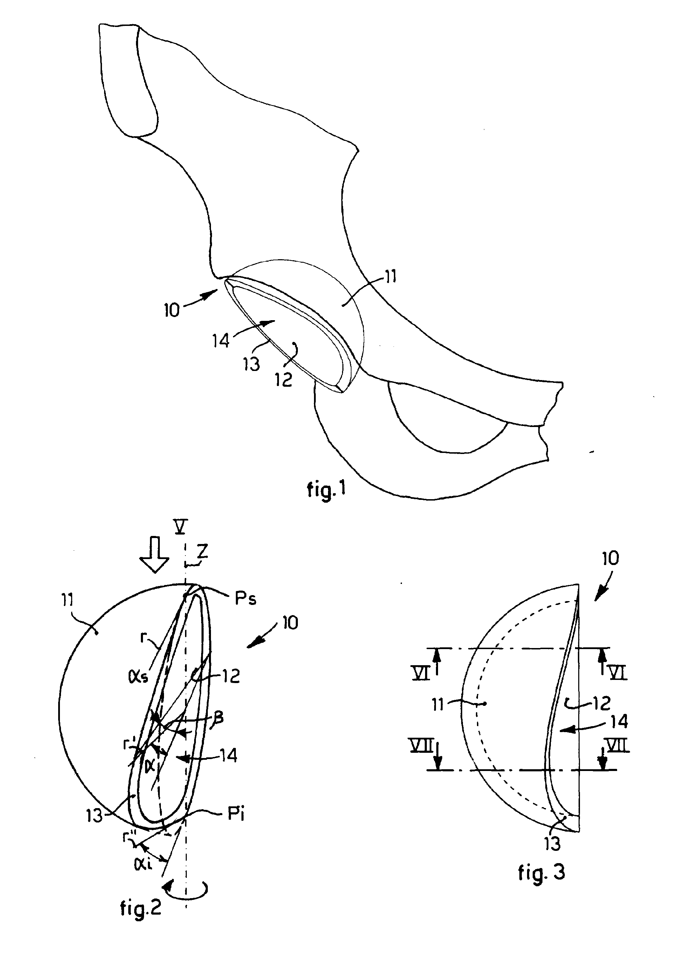

To be more exact, in fig. 2, r, r' and r" indicate three different positions of the straight line r in correspondence with, respectively, the upper pole Ps, the equator and the lower pole Pi.

In the preferential embodiment illustrated in fig. 2, in which the axis of roto-translation of r and the diameter Z of the cup coincide, the angle α corresponds, in geometrical terms, to the angle in the center of the angle of ante-version β.

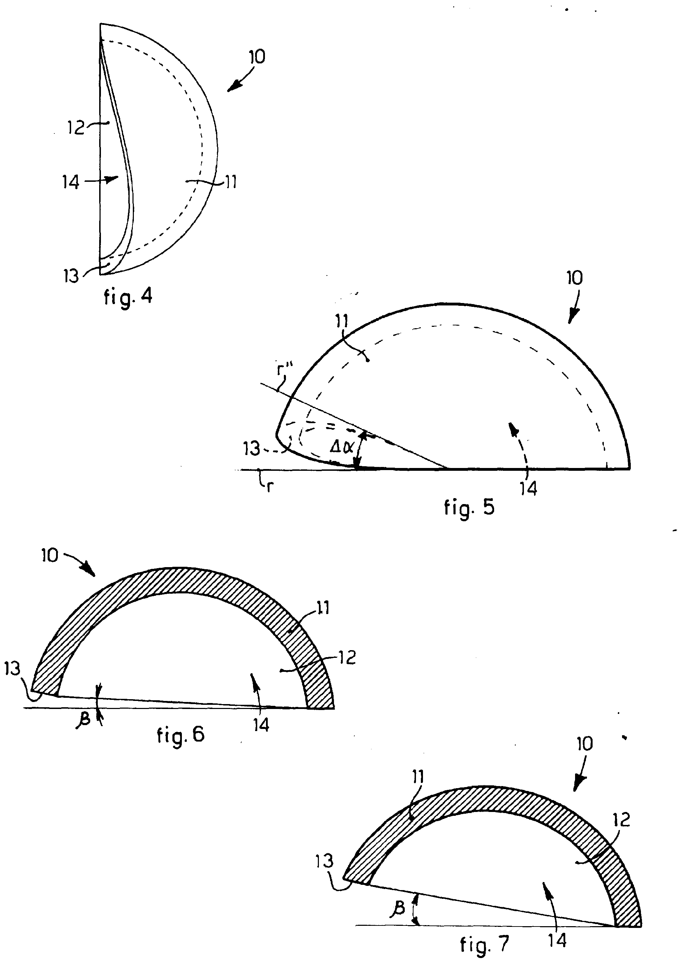

The absolute value of the overall rotation Δα is an angle comprised between about 10° and about 50°, although a value of about 25° is advised. The rotation can occur at a constant or variable angular speed, but a constant speed is advised. The rotation occurs in a clockwise direction in order to achieve the left cup 11 shown in figs. 2, 3, 5, 6 and 7, in an anti-clockwise direction to achieve the right cup 10 shown in figs. 1 and 4.

It is clear, however, that modifications and/or additions of parts may be made to the prosthetic acetabulum cup 10 as described heretofore, without departing from the field and scope of the present invention.

For example, it comes within the field of the present invention to provide that the axis of roto-translation PsPi, in the example coinciding with the vertical diameter Z, can be any straight line substantially parallel to the vertical diameter Z, and also inclined with respect thereto.

According to a variant, the thickness of the edge 13 of the cup 11 can be uniform or not, in this case having an eccentric outer surface with respect to the inner surface.

For example, according to another variant, the outer surface of the cup, instead of spherical as represented here, can be changed to ellipsoidal, truncated cone, etc.; it is also possible to add elements used to fix the bone (fins, holes for screws, etc...) .

It also comes within the field of the present invention to provide that the cup may be made in a single block or modular form, although the single block construction is recommended. It can also be made of any material held to be suitable, and can be covered externally with any material judged able to facilitate integration with the bone.

It is also clear that, although the present invention has been described with reference to specific examples, a person of skill in the art shall certainly be able to achieve many other equivalent forms of perfected acetabulum cup for arthro-prosthesis of the hip, having the characteristics as set forth in the claims and hence all coming within the field of protection defined thereby.

## Claims

1. Acetabulum cup for arthro-prosthesis of the hip comprising an inner surface (12) of a hemispherical conformation and including a shaped edge (13),
**characterized in that** said edge (13) has an anatomical and asymmetric profile different for the left and right sides with respect to a vertical diameter thereof (Z), so that an angle of ante-version (β) defined by said edge (13) varies from an upper end (Ps) to a lower end (Pi) of said vertical diameter (Z).

2. Cup as in claim 1, **characterized in that** said angle of ante-version (β) grows in a substantially progressive manner between the upper end and the lower end of said vertical diameter (Z).

3. Cup as in claim 1 or 2, **characterized in that** the sections normal for said vertical diameter (Z), made by proceeding from said upper end (Ps) to said lower end (Pi) of said vertical diameter (Z), identify arches whose subtended chord is rotated by said angle of ante-version (β) in an anti-clockwise direction for a right cup (10), and clockwise for a left cup (10).

4. Cup as in any claim hereinbefore, **characterized in that** said inner surface (12) is geometrically generated by sectioning a spherical cap with a straight line (r, r', r") which performs a roto-translation around said vertical diameter (Z).

5. Cup as in any claim from 1 to 3, **characterized in that** said inner surface (12) is geometrically generated by sectioning a spherical cap with a straight line (r, r', r") which performs a roto-translation around an axis substantially parallel or inclined with respect to said vertical diameter (Z).

6. Cup as in claims 4 and 5, **characterized in that** the rotation of said straight line (r, r', r") occurs in an anti-clockwise direction for a right cup (10), and clockwise for a left cup (10).

7. Cup as in any claim from 4 to 6, **characterized in that** the rotation and translation of said straight line (r, r', r") occur at substantially constant speed.

8. Cup as in any claim from 4 to 7, **characterized in that** said straight line (r, r', r") is orthogonal to its axis of roto-translation.

9. Cup as in any claim hereinbefore, **characterized in that** the relative rotation of said straight line (r, r', r") between said upper end (Pₛ) and said lower end (Pᵢ) is equal, in absolute values, to an angle (Δα) having an amplitude comprised between about 10° and about 50°.

10. Cup as in claim 9, **characterized in that** said angle (Δα) is equal to about 25°.
